# EUROPEAN PATENT APPLICATION

(11) **EP 1 174 523 A2**
(43) Date of publication of application: **23.01.2002**
(21) Application number: 01116929.9
(22) Date of filing: 11.07.2001
(51) Int. Cl.: C12Q 1/70, C12Q 1/68

(54) **Method for detecting an HBV-derived nucleic acid target sequence**

(30) Priority: 18.07.2000 SG 200004041
(71) Applicant: Government of Republic of Singapore, Singapore 169854 (SG)
(72) Inventor: Chen, Wei Ning, 22-114 Singapore 080104 (SG); Oon, Chong Jin, Singapore 266914 (SG)
(74) Representative: Brown, John David

(57) **Abstract**

The present invention relates generally to a nucleic acid-based assay to detect the presence of a viral pathogen and, in particular, hepatitis B virus. More particularly, the present invention provides a single-step amplification assay to detect hepatitis B viral nucleic acid sequences. The assay of the present invention is readily adaptable for automation and permits the rapid through-put of samples to be tested. The present invention further provides agents useful for performing a nucleic acid-based detection assay for hepatitis B virus and a kit comprising said agents.

SEQUENCE LISTING

<110> OON, Chong J

<120> DIAGNOSTIC ASSAY

<130> 2269257/EJH

<140>
<141>

<160> 3

<170> PatentIn Ver. 2.1

<210> 1
<211> 21
<212> DNA
<213> Artificial Sequence

<220>
<223> Description of Artificial Sequence:primer

<400> 1
caaggtatgt tgcccgtttg t                                                21

<210> 2
<211> 23
<212> DNA
<213> Artificial Sequence

<220>
<223> Description of Artificial Sequence:primer

<400> 2
tggctcagtt tactagtgcc att                                              23

<210> 3

<211> 6

<212> DNA

<213> Artificial Sequence

<220>

<223> Description of Artificial Sequence:primer

<400> 3

gaattc 6

## Description

### FIELD OF THE INVENTION

The present invention relates generally to a nucleic acid-based assay to detect the presence of a viral pathogen and, in particular, hepatitis B virus. More particularly, the present invention provides a single-step amplification assay to detect hepatitis B viral nucleic acid sequences. The assay of the present invention is readily adaptable for automation and permits the rapid through-put of samples to be tested. The present invention further provides agents useful for performing a nucleic acid-based detection assay for hepatitis B virus and a kit comprising said agents.

### BACKGROUND OF THE INVENTION

Bibliographic details of the publications numerically referred to in this specification are collected at the end of the description.

Hepatitis B virus (hereinafter referred to as "HBV") can cause debilitating disease conditions and can lead to acute liver failure, bleeding from cirrhosis and primary liver cancer. HBV infects millions of individuals annually and contributes to at least a million deaths each year.

HBV is a DNA virus which replicates *via* RNA intermediates and utilizes reverse transcription in its replication strategy. The HBV genome is of a complex nature having a partially double stranded DNA structure with overlapping open reading frames encoding various viral proteins.

Despite the availability of an HBV vaccine, hundreds of millions of individuals are carriers of the virus. HBV is generally identified by detection of the HBV surface antigen (HBsAg), the viral core antigen (HBcAg) and antibodies to HBeAg (anti-HBeAg).

Following infection with HBV or vaccination with HBsAg, serum antibodies appear to HBsAg (anti-HBs). This has been the basis for the immune-based detection of HBsAg. In this detection system, either HBsAg or anti-HBs are screened. The assay has been used to screen blood prior to transfusion for HBV, to detect HBV-related liver disease in patients with acute and chronic hepatitis, liver cirrhosis and primary liver cancer and screening for HBV carriers such as in transplantation recipients and donors.

The HBsAg comprises an antigenic region referred to as the "a" determinant (1). The "a" determinant is complex, conformational and dependent upon disulphide bonding among highly conserved cysteine residues. Genetic variation leading to changes in the "a" determinant has been implicated in mutants of HBV which escape the immunological response generated to conventional vaccines (2-6). One particularly common mutation is a glycine (G) to arginine (R) substitution at amino acid position 145 (G145F) of HBsAg. This mutation affects the "a" epitope region.

The increasing reliance on chemical and immunological intervention in treating or preventing HBV infection is resulting in greater selective pressure for the emergence of variants of HBV which are resistant to the interventionist therapy. Due to the overlapping genomic structure of HBV, HBV variants, may be directly or indirectly selected for by the use of chemical agents or vaccines.

Consequently, conventional antibody-based assays for HBsAg can result in false negative results. This can have very serious implications in terms of controlling spread of the disease and in patient management.

In work leading up to the present invention, the inventors sought an alternative assay for HBV. In accordance with the present invention, the inventors have developed a single-step nucleic acid amplification assay to screen for HBV nucleic acid sequences from HBV which may or may not display immuno-detectable viral markers. The nucleic acid-based assay of the present invention provides a sensitive assay for HBV agents especially where such agents may not be detectable by conventional immuno-based diagnostic tests. Furthermore, the assay of the present invention permits the development of specific immunoglobulin (e.g. IgG) and vaccines based on conserved regions amongst HBV variants.

### SUMMARY OF THE INVENTION

Throughout this specification, unless the context requires otherwise, the word "comprise", or variations such as "comprises" or "comprising", will be understood to imply the inclusion of a stated element or integer or group of elements or integers but not the exclusion of any other element or integer or group of elements or integers.

One aspect of the present invention provides a method for detecting an HBV-derived nucleic acid target sequence in a sample, said method comprising subjecting the sample to denaturing conditions to yield single stranded forms of said target sequence, contacting the denatured sample with a set of primers comprising at least two primers wherein at least one primer is capable of hybridizing to one strand of said target sequence and wherein at least one other primer is capable of hybridizing to a strand complementary to said first mentioned strand and wherein said primers are extendable from their 3' termini to form an extension product complementary to the strand to which each of said primers has hybridized and subjecting the sample to conditions to facilitate amplification to generate an amplified product comprising complementary extension products and then detecting for the presence of the amplification product wherein the presence of said amplified product is indicative of said HBV-derived nucleic acid target sequence.

Another aspect of the present invention contemplates a method for detecting an HBV-derived nucleic acid target sequence in a sample, said method comprising subjecting the sample to denaturing conditions to yield single stranded forms of said target sequence, contacting the denatured sample with a set of primers comprising at least two primers wherein at least one primer is capable of hybridizing to one strand of said target sequence and wherein at least one other primer is capable of hybridizing to a strand complementary to said first strand and wherein said primers are extendable from their 3' termini to form an extension product complementary to the strand to which each of said primer has hybridized and wherein the primers are selected to hybridize to regions corresponding to or flanking a conserved nucleotide sequence within or part of the nucleotide sequence encoding the HBsAg, and subjecting the sample to conditions to facilitate amplification to generate an amplified product comprising complementary extension products and then detecting for the presence of the amplification product wherein the presence of said amplification product is indicative of said HBV-derived nucleic acid target sequence.

Yet another aspect of the present invention contemplates a method for detecting an HBV-derived DNA target sequence in a sample, said method comprising optionally subjecting said sample to reverse transcription conditions to yield single or double stranded cDNA molecules from HBV-derived mRNA, subjecting the sample to denaturing conditions to yield single stranded DNA molecules corresponding to said target sequence, contacting the denatured sample with a set of primers comprising at least two primers wherein at least one primer is capable of hybridizing to one DNA strand of said target sequence and wherein at least one other primer is capable of hybridizing to a strand complementary to said first mentioned strand and wherein said primers are extendable from their 3' termini to form an extension product complementary to the strand to which each of said primers has hybridized and subjecting the sample to amplification conditions to facilitate amplification to generate an amplified product comprising complementary extension products and then detecting the amplification product wherein the presence of said amplification product is indicative of said HBV-derived DNA target sequence.

Still another aspect of the present invention contemplates a method for detecting an HBV-derived DNA target sequence in a sample, said method comprising optionally subjecting said sample to reverse transcription conditions to yield single or double stranded cDNA molecules from HBV-derived mRNA, subjecting the sample to denaturing conditions to yield single stranded DNA molecules corresponding to said target sequence, contacting the denatured sample with a set of primers comprising at least two primers wherein at least one primer is capable of hybridizing to one DNA strand of said target sequence and wherein at least one other primer is capable of hybridizing to a strand complementary to said first mentioned strand and wherein said primers are extendable from their 3' termini to form an extension product complementary to the strand to which each of said primers has hybridized wherein one of said primers is labelled with a reporter molecule capable of giving an identifiable signal and the other of said primers is labelled with a capturable moiety, subjecting said sample to conditions to facilitate amplification to generate an amplification product comprising complementary extension products having a reporter molecule capable of providing an identified signal on one strand and a capturable moiety to a solid support on another strand, immobilizing the amplification product *via* its capturable moiety and then screening for the detectable signal wherein the presence of the signal is indicative of amplification product and thereby HBV-derived target DNA sequence.

Still yet another aspect of the present invention contemplates a method for detecting an HBV-derived DNA target sequence in a sample, said method comprising introducing said sample to a reaction vessel having a primer immobilized to a solid support and a second primer in solution phase wherein both primers are capable of hybridizing to a complementary nucleotide sequence on complementary single strands of HBV-derived DNA in a region within, proximal or adjacent to a conserved region on the HBV genome and wherein the solution phase primer is labelled with a reporter molecule capable of giving an identifiable signal, subjecting the reaction vessel to conditions to facilitate amplification and then detecting the presence of the identifiable signal wherein the presence of said signal is indicative of the presence of HBV-derived DNA.

Even still another aspect of the present invention is directed to a method for detecting one or more HBV-derived DNA target sequences from the same or different strains or variants of HBV, said method comprising contacting a sample putatively comprising HBV-derived DNA in single stranded form with two or more primers immobilized individually or in an array to a solid support in a reaction vessel wherein the reaction vessel further comprises solution phase primers having an partner immobilized to the solid support wherein the immobilized primer and its solution phase partner primer are capable of amplifying under amplifying conditions a region of HBV-derived DNA wherein the solution phase primer carries a reporter molecule capable of giving an identifiable signal such that the presence of a signal at a defined location on the solid support enables the identification of a particular HBV isolate or variant.

Another aspect of the present invention provides an array of two or more oligonucleotide primers immobilized to a solid support, said primers capable of hybridizing to a complementary nucleotide sequence from HBV-derived DNA.

Yet another aspect of the present invention provides for the use of a matrix comprising an array of oligonucleotides defined by the formula a₁a₂ ... aₙ, having coordinates on the matrix (x₁y₁), (x₂y₂) ... (xₙyₙ) wherein a₁a₂ ... aₙ represent the same or different oligonucleotides totalling n oligonucleotides and each oligonucleotide is definable by grid coordinates (x₁y₁), (x₂y₂) ... (xₙyₙ) and wherein the oligonucleotides have amplification partners defined by b₁b₁ ... bₙ such that oligonucleotides a₁b₁, a₂b₂ ... aₙbₙ are capable of hybridizing to complementary strands of HBV-derived DNA wherein the intervening DNA comprises a sequence of nucleotides conserved between two or more HBV variants, such as HBsAg variants, wherein said matrix is useful for detecting particular HBV agents defined by carrying the conserved amplification region of said HBV-derived DNA.

### BRIEF DESCRIPTION OF THE FIGURES

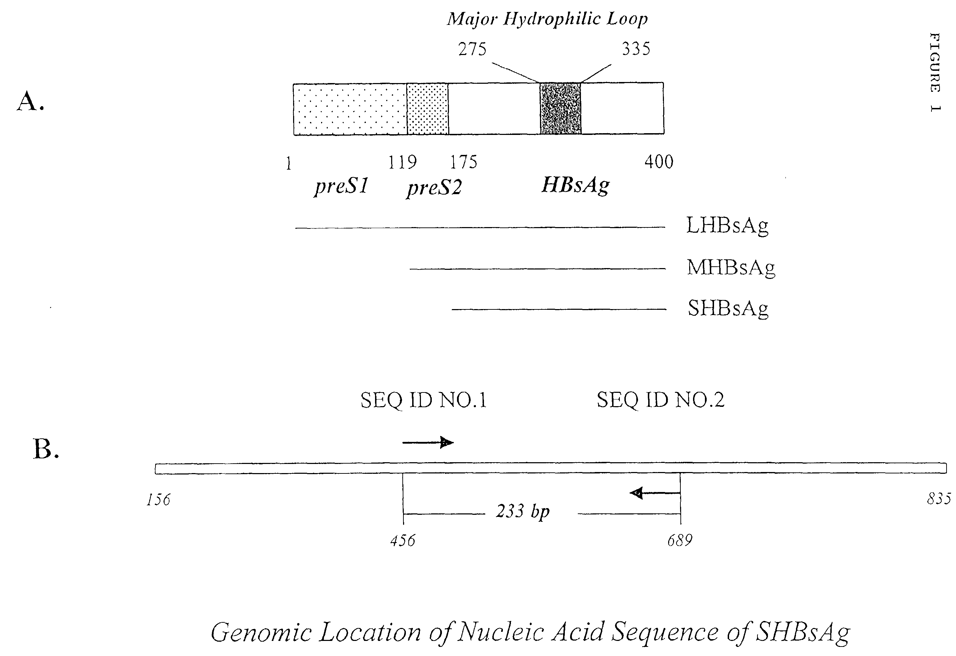

**Figure 1** is a diagrammatic representation showing the structural organization of HBV surface antigen. (A) The localization of the major hydrophilic loop covering the conserved "a" determinant (adapted from Chen *et al*. (7)). The size of the full-length HBsAg in amino acid residues is indicated (1-400). Positions of preS1 (1-118), preS2 (119-174) and the major HBsAg (175-400) are indicated below the box. The position of the major hydrophilic loop in relation to the full-length HBsAg is also indicated. The corresponding position of the major hydrophilic loop within the major HBsAg (SHBsAg) is from amino acid 100 to 160, and the conserved "a" determinant from 124 to 147 within SHBsAg. (B) The genomic location of the region coding for SHBsAg. The positions of 5'- and 3'-end of the coding region are indicated below the box (nt 156 and 835 of the HBV genome respectively, the position 1 being defined as the first A nucleotide of the Eco*RI* site-GAATTC (<400>3) of the wild-type HBV with Z35717 as its GenBank accession number). The positions of the primer oligonucleotides provided in the present invention (<400>1 and <400>2) are also indicated below the box (starting from nt 456 and 689 of the HBV genome, respectively). The size of PCR-amplified DNA fragment utilizing the primer oligonucleotides of the present invention (<400>1 and <400>2) is 233 base pairs (bp), as indicated below the box.

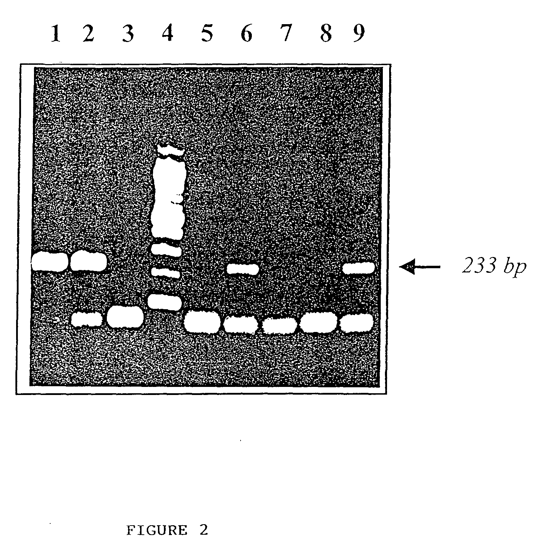

**Figure 2** is a photographic representation showing the electrophoresis pattern of PCR-amplified HBV fragment from test samples, negative for HBsAg by immune-based diagnostic kits but positive for anti-HBc and anti-HBs, utilizing the primer oligonucleotides provided in the present invention (<400>1 and <400>2). Indicated in *lane 4* are migration positions of molecular size markers (100 bp ladder, MBI Fermentas). *Lane 1* corresponds to the first test sample in Table 1; *lane 2* corresponds to the second test sample in Table 1. *Lanes 6 and 9* correspond to the third and fourth test samples in Table 1, respectively. No amplification products are seen in *lanes 3*, *5*, *7* and *8* that represent test samples displaying similar viral markers (negative for HBsAg, positive for anti-HBc and anti-HBs).

### DETAILED DESCRIPTION OF THE PREFERRED EMBODIMENTS

The present invention is predicated in part on the identification of nucleotide sequences which are conserved amongst HBV variants and their use to develop primers for amplification-based diagnostic assays for HBV.

Several terms are defined below for clarification purposes.

"Amplification mixture" refers to an aqueous solution comprising the various reagents required to amplify a target viral-derived nucleic acid sequence. These include: enzyme, aqueous buffers, salts, target nucleic acid and deoxynucleoside triphosphates.

"Amplification system" refers to any *in vitro* means for multiplying the copies of a target sequence of nucleic acid.

"Amplification reagents" refer to the various buffers, enzymes, primers and deoxynucleoside triphosphates required for amplification.

"Nucleic acid" refers to a ribonucleotide or deoxyribonucleotide polymer in either single or double stranded form.

"Polymerase" refers to enzymes able to catalyze the synthesis of DNA from nucleoside triphosphate precursors. In the PCR amplification of the present invention, the polymerases are preferably template-dependent, thermostable and typically add nucleotides to the 3'-end of the DNA polymer being elongated.

"Oligonucleotide" refers to a molecule comprised of two or more ribonucleotides or deoxyribonucleotides.

"Primer" refers to an oligonucleotide, whether natural or synthetic, capable of acting a s a point of initiation of DNA synthesis under conditions in which synthesis of a primer extension product that is complementary to a target nucleic acid strand is induced. These conditions include four different nucleoside triphosphates and polymerase in an appropriate buffer and at a suitable temperature. A primer is preferably a single stranded oligodeoxynucleotide. The appropriate length of a primer depends on the purpose and conditions of use of the primer (including sequence conservation of the target nucleic acid, PCR cycling conditions and the composition of the primer).

The present invention provides a method for detecting an HBV-derived nucleic acid target sequence in a sample, said method comprising subjecting the sample to denaturing conditions to yield single stranded forms of said target sequence, contacting the denatured sample with a set of primers comprising at least two primers wherein at least one primer is capable of hybridizing to one strand of said target sequence and wherein at least one other primer is capable of hybridizing to a strand complementary to said first mentioned strand and wherein said primers are extendable from their 3' termini to form an extension product complementary to the strand to which each of said primers has hybridized and subjecting the sample to conditions to facilitate amplification to generate an amplified product comprising complementary extension products and then detecting for the presence of the amplification product wherein the presence of said amplified product is indicative of said HBV-derived nucleic acid target sequence.

In one embodiment, the target sequence corresponds to a region on the HBV genome which is conserved amongst HBV variants and, in particular, HBV variants having altered HBsAg's. An altered HBsAg may comprise a single or multiple amino acid substitution, deletion and/or addition resulting from a single or multiple nuclcotide substitution, deletion and/or addition in the nucleotide sequence encoding the HBsAg. A conserved region in the HBV genome and, in particular, within the nucleotide sequence encoding HBsAg is proposed to remain relatively stable despite immunological changes to the HBsAg. Consequently, HBV variants which may no longer be detected in an immune-based HBsAg assay are proposed, in accordance with the present invention, to be still detectable *via* a conserved region in the HBsAg gene. The present invention extends, however, to the use of the subject method to identifying variable regions of the HBV genome such as those resulting in modified or new epitopes (e.g. on HBsAg).

Reference to HBsAg variants also encompasses variants in other overlapping genes such as the DNA polymerase.

This and other aspects of the present invention are not to be limited by the term "gene" which is encompassed by terms such as "nucleic acid molecule encoding", "nucleotide sequence encoding" and "genetic sequence encoding".

The term "gene" is used in its broadest sense and includes cDNA corresponding to the exons of a gene. Accordingly, reference herein to a "gene" is to be taken to include:-
(i) a classical genomic gene consisting of transcriptional and/or translational regulatory sequences and/or a coding region and/or non-translated sequences (i.e. introns, 5'- and 3'- untranslated sequences); or
(ii) mRNA or cDNA corresponding to the coding regions (i.e. exons) and 5'- and 3'-untranslated sequences of the gene.

The term "gene" is also used to describe synthetic or fusion molecules encoding all or part of an expression product. In particular embodiments, the term "nucleic acid molecule" and "gene" may be used interchangeably.

Accordingly, another aspect of the present invention contemplates a method for detecting an HBV-derived nucleic acid target sequence in a sample, said method comprising subjecting the sample to denaturing conditions to yield single stranded forms of said target sequence, contacting the denatured sample with a set of primers comprising at least two primers wherein at least one primer is capable of hybridizing to one strand of said target sequence and wherein at least one other primer is capable of hybridizing to a strand complementary to said first strand and wherein said primers are extendable from their 3' termini to form an extension product complementary to the strand to which each of said primer has hybridized and wherein the primers are selected to hybridize to regions corresponding to or flanking a conserved nucleotide sequence within or part of the nucleotide sequence encoding the HBsAg, and subjecting the sample to conditions to facilitate amplification to generate an amplified product comprising complementary extension products and then detecting for the presence of the amplification product wherein the presence of said amplification product is indicative of said HBV-derived nucleic acid target sequence.

A sample is generally, but not exclusively, a biological sample comprising tissue or tissue extract, fluid including blood or excretia or any other material which potentially comprises HBV particles or HBV DNA, mRNA or RNA replicative intermediates. A first step would generally comprise, therefore, isolating a sample from a mammalian subject including, in a preferred embodiment, a human subject . The sample may also be an environmental sample or a sample from a potential source of HBV contamination.

Although the present invention is particularly directed to "escape" HBV variants, i.e. variants to which antibodies to at least one form of HBsAg no longer substantially interact, the present invention further extends to any region of the HBV genome which is conserved amongst HBV variants and, in particular, clinically relevant HBV variants and which serves as a useful target sequence. Particularly useful regions contemplated on the HBV genome are those which encode new epitopes or HBsAg. Such regions are useful for generating recombinant peptides for diagnostic purposes.

The present invention provides, therefore, a means for detecting an HBV by detecting HBV-derived nucleic acid sequences. An "HBV-derived" nucleic acid sequence includes the HBV genome itself, single stranded DNA forms thereof, RNA replicative intermediates as well as cDNA single or double stranded molecules prepared using a reverse transcriptase. With respect to the latter, a mRNA transcript from an HBV-derived nucleotide sequence may be subject to reverse transcription to produce a complementary DNA strand. The latter strand and/or its DNA complement may then be the subject of the amplification reaction. The detection of RNA replicative intermediates and/or cDNA corresponding to HBV-derived mRNA is also an indication of active viral replication.

Any form of amplification reaction may be used in the practice of the present invention including but not limited to the polymerase chain reaction (PCR), ligation chain reaction, nucleic acid sequence based amplification, Q replicase based amplification, strand displacement method, rolling circle amplification and recirculating allele-specific primer extension. Preferably, however, PCR is employed.

Accordingly, another aspect of the present invention contemplates a method for detecting an HBV-derived DNA target sequence in a sample, said method comprising optionally subjecting said sample to reverse transcription conditions to yield single or double stranded cDNA molecules from HBV-derived mRNA, subjecting the sample to denaturing conditions to yield single stranded DNA molecules corresponding to said target sequence, contacting the denatured sample with a set of primers comprising at least two primers wherein at least one primer is capable of hybridizing to one DNA strand of said target sequence and wherein at least one other primer is capable of hybridizing to a strand complementary to said first mentioned strand and wherein said primers are extendable from their 3' termini to form an extension product complementary to the strand to which each of said primers has hybridized and subjecting the sample to amplification conditions to facilitate amplification to generate an amplified product comprising complementary extension products and then detecting the amplification product wherein the presence of said amplification product is indicative of said HBV-derived DNA target sequence.

In a particularly preferred embodiment, the primers are selected from within the nucleotide sequence encoding HBsAg. Most preferably, the primers are <400>1 [sense primer] and <400>2 [antisense primer]. The present invention also extends to oligonucleotide primers having at least about 70% similarity to <400>1 or <400>2 as well as primers capable of hybridizing to <400>1 or <400>2 or their complements under low stringency conditions.

The term "similarity" as used herein includes exact identity between compared sequences at the nucleotide level. Where there is non-identity at the nucleotide level, "similarity" includes differences between sequences which result in different amino acids that are nevertheless related to each other at the structural, functional, biochemical and/or conformational levels. In a particularly preferred embodiment, nucleotide sequence comparisons are made at the level of identity rather than similarity.

Terms used to describe sequence relationships between two or more polynucleotides or polypeptides include "reference sequence", "comparison window", "sequence similarity", "sequence identity", "percentage of sequence similarity", "percentage of sequence identity", "substantially similar" and "substantial identity". A "reference sequence" is at least 12 but frequently 15 to 18 and often at least 25 or above, such as 30 monomer units, inclusive of nucleotides and amino acid residues, in length. Because two polynucleotides may each comprise (1) a sequence (i.e. only a portion of the complete polynucleotide sequence) that is similar between the two polynucleotides, and (2) a sequence that is divergent between the two polynucleotides, sequence comparisons between two (or more) polynucleotides are typically performed by comparing sequences of the two polynucleotides over a "comparison window" to identify and compare local regions of sequence similarity. A "comparison window" refers to a conceptual segment of typically 12 contiguous residues that is compared to a reference sequence. The comparison window may comprise additions or deletions (i.e. gaps) of about 20% or less as compared to the reference sequence (which does not comprise additions or deletions) for optimal alignment of the two sequences. Optimal alignment of sequences for aligning a comparison window may be conducted by computerised implementations of algorithms (GAP, BESTFIT, FASTA, and TFASTA in the Wisconsin Genetics Software Package Release 7.0, Genetics Computer Group, 575 Science Drive Madison, WI, USA) or by inspection and the best alignment (i.e. resulting in the highest percentage homology over the comparison window) generated by any of the various methods selected. Reference also may be made to the BLAST family of programs as for example disclosed by Altschul *et al*. (8). A detailed discussion of sequence analysis can be found in Unit 19.3 of Ausubel *et al*. (9).

The terms "sequence similarity" and "sequence identity" as used herein refers to the extent that sequences are identical or functionally or structurally similar on a nucleotide-by-nucleotide basis over a window of comparison. Thus, a "percentage of sequence identity", for example, is calculated by comparing two optimally aligned sequences over the window of comparison, determining the number of positions at which the identical nucleic acid base (e.g. A, T, C, G, I) occurs in both sequences to yield the number of matched positions, dividing the number of matched positions by the total number of positions in the window of comparison (i.e., the window size), and multiplying the result by 100 to yield the percentage of sequence identity. For the purposes of the present invention, "sequence identity" will be understood to mean the "match percentage" calculated by the DNASIS computer program (Version 2.5 for windows; available from Hitachi Software engineering Co., Ltd., South San Francisco, California, USA) using standard defaults as used in the reference manual accompanying the software. Similar comments apply in relation to sequence similarity.

Reference herein to a low stringency includes and encompasses from at least about 0 to at least about 15% v/v formamide and from at least about 1 M to at least about 2 M salt for hybridization, and at least about 1 M to at least about 2 M salt for washing conditions. Generally, low stringency is at from about 25-30°C to about 42°C. The temperature may be altered and higher temperatures used to replace formamide and/or to give alternative stringency conditions. Alternative stringency conditions may be applied where necessary, such as medium stringency, which includes and encompasses from at least about 16% v/v to at least about 30% v/v formamide and from at least about 0.5 M to at least about 0.9 M salt for hybridization, and at least about 0.5 M to at least about 0.9 M salt for washing conditions, or high stringency, which includes and encompasses from at least about 31% v/v to at least about 50% v/v formamide and from at least about 0.01 M to at least about 0.15 M salt for hybridization, and at least about 0.01 M to at least about 0.15 M salt for washing conditions. In general, washing is carried out Tₘ = 69.3 + 0.41 (G+C)% (10). However, the Tₘ of a duplex DNA decreases by 1°C with every increase of 1% in the number of mismatch base pairs (11). Formamide is optional in these hybridization conditions. Accordingly, particularly preferred levels of stringency are defined as follows: low stringency is 6 x SSC buffer, 0.1% w/v SDS at 25-42°C; a moderate stringency is 2 x SSC buffer, 0.1% w/v SDS at a temperature in the range 20°C to 65°C; high stringency is 0.1 x SSC buffer, 0.1% w/v SDS at a temperature of at least 65°C.

Reference herein to a "primer" is not to be taken as any limitation as to structure, size or function. The primer may be used as an amplification molecule or may be used as a probe for hybridization purposes. The preferred form of the molecule is as a nucleic acid primer for amplification.

Reference herein to a "nucleic acid primer" includes reference to a sequence of deoxyribonucleotides or ribonucleotides comprising at least 3 nucleotides. Generally, the nucleic acid primer comprises from about 3 to about 100 nucleotides, preferably from about 5 to about 50 nucleotides and even more preferably from about 5 to about 25 nucleotides. A primer having less than 50 nucleotides may also be referred to herein as an "oligonucleotide primer". The primers of the present invention may be synthetically produced by, for example, the stepwise addition of nucleotides or may be fragments, parts, portions or extension products of other nucleotide acid molecules. The term "primer" is used in its most general sense to include any length of nucleotides which, when used for amplification purposes, can provide a free 3' hydroxyl group for the initiation of DNA synthesis by a DNA polymerase. DNA synthesis results in the extension of the primer to produce a primer extension product complementary to the nucleic acid strand to which the primer has hybridized.

The extension of the hybridized primer to produce an extension product is included herein by the term "amplification". Amplification generally occurs in cycles of denaturation followed by primer hybridization and extension. The present invention encompasses from about 1 cycle to about 120 cycles, preferably from about 2 to about 70 cycles and even more preferably from about 5 to about 40 cycles including about 10, 15, 20, 25 and 30 cycles.

The amplification product (also referred to as an amplimer) may be detected by any convenient means. In one embodiment, the products of amplification are separated by electrophoresis and then visualized using, for example, ultra-violet light after ethidium bromide staining. Alternatively, the amplification products may be resolved using various forms of chromatography such as HPLC or spectroscopy such as MALDI-TOF and mass spectrometry. Yet another alternative uses primers labelled with a reporter molecule. With respect to the latter, one of the primers may be labelled with a reporter molecule while the other of the primers may be labelled with a capturing moiety to anchor amplimers to a solid support which are then identified *via* the reporter signal. This embodiment may be practised with the set of primers used for amplification or with a second, nested set of primers. In a preferred embodiment, however, only a single set of primers is employed such that the assay is a single step amplification assay.

Accordingly, another aspect of the present invention contemplates a method for detecting an HBV-derived DNA target sequence in a sample, said method comprising optionally subjecting said sample to reverse transcription conditions to yield single or double stranded cDNA molecules from HBV-derived mRNA, subjecting the sample to denaturing conditions to yield single stranded DNA molecules corresponding to said target sequence, contacting the denatured sample with a set of primers comprising at least two primers wherein at least one primer is capable of hybridizing to one DNA strand of said target sequence and wherein at least one other primer is capable of hybridizing to a strand complementary to said first mentioned strand and wherein said primers are extendable from their 3' termini to form an extension product complementary to the strand to which each of said primers has hybridized wherein one of said primers is labelled with a reporter molecule capable of giving an identifiable signal and the other of said primers is labelled with a capturable moiety, subjecting said sample to conditions to facilitate amplification to generate an amplification product comprising complementary extension products having a reporter molecule capable of providing an identifiable signal on one strand and a capturable moiety to a solid support on another strand, immobilizing the amplification product *via* its capturable moiety and then screening for the detectable signal wherein the presence of the signal is indicative of amplification product and thereby HBV-derived target DNA sequence.

Many variations to the above-mentioned assay will be apparent to the skilled artisan. For example, the amplification reaction may occur in a vessel or microtitre well having a capture molecule immobilized to the walls of the vessel or well. The capture molecule may be, for example, a primer capable of hybridizing and capturing a nucleotide sequence within the amplimer or a nucleotide sequence complementary to the capture molecule on the capturable moiety of one of the primers. Alternatively, the capture molecule may be a DNA binding protein capable of specifically recognizing the capturable moiety of one of the primers. The capture molecule may also participate in the amplification reaction.

The capture molecule may be immobilized to the solid phase by any convenient means. The solid phase may be any structure having a surface which can be derivatized to anchor a nucleic acid primer or other capture molecule. Preferably, the solid phase is a planar material such as the side of a microtitre well or the side of a dipstick.

Solid supports contemplated herein are typically glass or a polymer, such as but not limited to cellulose, ceramic material, nitrocellulose, polyacrylamide, nylon, polystyrene and its derivatives, polyvinylidene difluoride (PVDF), methacrylate and its derivatives, polyvinyl chloride or polypropylene. Nitrocellulose is particularly useful. A solid support may also be a hybrid such as a nitrocellulose film supported on a glass or polymer matrix. Reference to a "hybrid" includes reference to a layered arrangement of two or more glass or polymer surfaces listed above. The solid support may be in the form of a membrane or tubes, beads, discs or microplates, dipsticks, microtitre tray wells or any other surface suitable for conducting the assay.

In one embodiment, the anchored nucleic acid captures a target nucleic acid molecule by hybridization and optionally participates in an amplification reaction. Alternatively, the anchored nucleic acid molecule captures amplified nucleic acid molecules.

Methods for linking nucleic acid molecules to solid supports are well known in the art. Processes for linking the primer to the solid phase include amide linkage, amidate linkage, thioether linkage and the introduction of amino groups on to the solid phase. Examples of linkage to a solid phase can be found in International Patent Application No. PCT/AU92/00587 [WO 93/09250].

The anchored primer may participate with a solution phase primer in an amplification reaction. Alternatively, a "generic" primer is anchored to the solid support in order to amplify the nucleic acid molecule comprising a target sequence. Specific amplification of the target sequence can then be achieved by solution phase primers

A range of labels providing a detectable signal may be employed. The label may be associated with a particular nucleic acid molecule or nucleotide or it may be attached to an intermediate which subsequently binds to a nucleic acid molecule or nucleotide.

The label may be selected from a group including a chromogen, a catalyst, an enzyme, a fluorophore, a luminescent molecule, a chemiluminescent molecule, a lanthanide ion such as Europium (Eu³⁴), a radioisotope and a direct visual label. In the case of a direct visual label, use may be made of a colloidal metallic or non-metallic particular, a dye particle, an enzyme or a substrate, an organic polymer, a latex particle, a liposome, or other vesicle containing a signal producing substance and the like. A large number of enzymes suitable for use as labels is disclosed in United States Patent Nos. 4,366,241, 4,843,000 and 4,849,338. Suitable enzyme labels useful in the present invention include alkaline phosphatase, horseradish peroxidase, luciferase, -galactosidase, glucose oxidase, lysozyme, malate dehydrogenase and the like. The enzyme label may be used alone or in combination with a second enzyme which is in solution. Alternatively, a fluorophore which may be used as a suitable label in accordance with the present invention includes, but is not limited to, fluorescein, rhodamine, Texas red, lucifer yellow or R-phycoerythrin.

In another embodiment, one of the primers is anchored to a solid support such as the wall of a vessel or microtitre well and the other of the primers is in the solution phase. The solution phase primer is generally labelled with a reporter molecule. After the amplification reaction, the presence of a detectable signal is indicative of an amplimer.

Accordingly, another aspect of the present invention contemplates a method for detecting an HBV-derived DNA target sequence in a sample, said method comprising introducing said sample to a reaction vessel having a primer immobilized to a solid support and a second primer in solution phase wherein both primers are capable of hybridizing to a complementary nucleotide sequence on complementary single strands of HBV-derived DNA in a region within, proximal or adjacent to a conserved region on the HBV genome and wherein the solution phase primer is labelled with a reporter molecule capable of giving an identifiable signal, subjecting the reaction vessel to conditions to facilitate amplification and then detecting the presence of the identifiable signal wherein the presence of said signal is indicative of the presence of HBV-derived DNA.

In yet another embodiment, the present invention employs an array of primers to different regions of the HBV genome such as conserved regions or conserved and variable regions on the HBV genome. This is particularly useful if the HBV is required to be "typed" or its variable region identified. In one useful embodiment, multiple primers directed to different regions of the HBV genome are immobilized to a solid support such as a microchip, reaction vessel wall, dipstick, slide or other matrix. The primers are generally arranged in an array with particular coordinates for identification of the primer. The term "array", however, is not to imply any particular order and the "array" may also comprise random spots within an overall pattern.

In any event, each immobilized primer would generally have a solution phase second primer such that particular regions of the HBV genome can be targeted for amplification. The solution phase primers generally carry a reporter molecule capable of giving an identifiable signal. Upon amplification, the presence of a signal in defined locations on the array can be used to type a particular HBV.

Accordingly, another aspect of the present invention is directed to a method for detecting one or more HBV-derived DNA target sequences from the same or different strains or variants of HBV, said method comprising contacting a sample putatively comprising HBV-derived DNA in single stranded form with two or more primers immobilized individually or in an array to a solid support in a reaction vessel wherein the reaction vessel further comprises solution phase primers having an partner immobilized to the solid support wherein the immobilized primer and its solution phase partner primer are capable of amplifying under amplifying conditions a region of HBV-derived DNA wherein the solution phase primer carries a reporter molecule capable of giving an identifiable signal such that the presence of a signal at a defined location on the solid support enables the identification of a particular HBV isolate or variant.

As stated above, the preferred primers are as set forth in <400>1 and <400>2 or primers having at least about 70% similarity to <400>1 or <400>2 as well as primers capable of hybridizing to <400>1 or <400>2 or their complements under low stringency conditions. Preferably, these are prepared by chemical synthesis, however, they may also be produced as fragments of nucleic acid molecules such as but not limited to restriction fragments. The primers are conveniently selected following sequence alignment of known HBV strains including variants such as those carrying mutations in the major hydrophilic loop of HBsAg. Where approximately 20 contiguous nucleotides arc shown to be conserved, then these are selected as primer candidates for conserved regions of the HBV genome.

Yet another aspect of the present invention provides, therefore, an array of two or more oligonucleotide primers immobolized to a solid support, said primers capable of hybridizing to a complementary nucleotide sequence from HBV-derived DNA.

Preferably, the region to which the primers hybridize is within, adjacent or proximal to a nucleotide sequence of from about 10 to about 50 nucleotides conserved amongst two or more HBsAg variants.

Preferably, the array is in the form of a biochip or other micro-matrix, the wall of a microtitre well, slide, dipstick or other suitable matrix.

The array may also be adapted for analysis by a computer program which is capable of detecting the presence of immobilized amplimers providing a detectable signal.

Accordingly, another aspect of the present invention contemplates a computer program-assisted method for detecting or identifying HBV-derived nucleic acid sequences, said method comprising:-
(i) means to perform amplification reaction using a set of primers comprising at least two primers wherein at least one primer is capable of hybridizing to one strand of a target sequence and wherein at least one other primer is capable of hybridizing to a strand complementary to said first mentioned strand; and
(ii) data processing means to record the presence of an identifiable signal.

Yet another aspect of the present invention provides for the use of a matrix comprising an array of oligonucleotides defined by the formula a₁a₂ ... aₙ, having coordinates on the matrix (x₁y₁), (x₂y₂) ... (xₙyₙ) wherein a₁a₂ ... aₙ represent the same or different oligonucleotides totalling n oligonucleotides and each oligonucleotide is definable by grid coordinates (x₁y₁), (x₂y₂) ... (xₙyₙ) and wherein the oligonucleotides have amplification partners defined by b₁b₁ ... bₙ such that oligonucleotides a₁b₁, a₂b₂ ... aₙbₙ are capable of hybridizing to complementary strands of HBV-derived DNA wherein the intervening DNA comprises a sequence of nucleotides conserved between two or more HBV variants, such as HBsAg variants, wherein the matrix is useful for detecting particular HBV agents defined by carrying the conserved amplification region of HBV genome.

The primer oligonucleolides of the present invention arc utilized in conjunction with an amplification reaction such as PCR of the target HBV nucleic acid. Although the amplification processes are well known in the art, some general information on PCR procedures used in accordance with the present invention is highlighted below for clarification purposes.

To amplify a target HBV nucleic acid sequence in a test sample, the sequence is required to be accessible to the components of the amplification system. This is achieved by isolating the HBV target nucleic acid from the sample prior to PCR amplification. Techniques for extracting nucleic acid molecules from biological samples are well established and widely used (12).

The first step of each cycle of the PCR amplification involves the separation of the HBV nucleic acid duplex, for example, through heating the sample at a suitable temperature such as 95°C. Once the strands are separated, the next step in PCR involves hybridizing the separated strands with primers which flank the target sequence. The primers are then extended to form complementary copies of the target strands. For PCR amplification, the primers are designed so that the position at which each primer hybridizes along a duplex sequence is such that an extension product synthesized from one primer, when separated from the template, serves as a template for the extension of the other primer. The cycle of denaturation, hybridization and extension is repeated for from one cycle to about 120 cycles but generally about 35 cycles to minimize the number of mutations introduced to the amplified product through the PCR amplification.

Template-dependent extension of primers in PCR amplification is catalyzed by a polymerizing agent in the presence of adequate amounts of four deoxyribonucleoside triphosphates (typically dATP, dGTP, dCTP and dTTP) in a reaction medium comprising the appropriate salts, metal cations and pH buffering system. Suitable polymerizing agents are enzymes known to catalyze template-dependent DNA synthesis. These include Taq polymerase, a heat-stable DNA polymerase isolated from *Thermus aquaticus* and high fidelity Pfu polymerase. The latter enzyme is widely used in the high precision amplifications. The PCR amplification is usually carried out as an automated process with a thermostable enzyme. In this process, the temperature of the reaction mixture is cycled through a denaturing phase, a primer annealing phase and an extension reaction phase.

Experimental measures are taken to avoid the contamination of a PCR amplification by the amplified nucleic acid from other reactions and non-specific amplification. To avoid the contamination from the other reactions, each reaction mixture comprising the appropriate salts, metal cations and pH buffering system, adequate amounts of four deoxyribonucleoside triphosphates (typically dATP, dGTP, dCTP and dTTP), DNA polymerase and primer oligonucleotides, but excluding the viral target nucleic acid isolated from a biological sample, is subjected to UV irradiation for 5 minutes at 260 nm. This treatment eliminates all potential double stranded DNA molecules in the pre-amplification reaction mixture, while keeping the single stranded primer oligonucleotides and other above-mentioned components of the reaction mixture intact. On the other hand, sequencing analysis of the amplified viral nucleic acid will not only eliminate the cases of contamination by non-specific amplifications, but also determine the nature of the amplified products, in particular the potential mutations on the major hydrophilic loop that result in the viral escape from detection using current immuno-based diagnostic assays.

The present invention encompasses, therefore, novel HBV variants such as those identified in accordance with the method of the present invention.

Accordingly, another aspect of the present invention provides an HBV variant, generally in isolated form, which HBV variant is identified by a method of detecting an HBV-derived nucleic acid target sequence, said method comprising subjecting the sample to denaturing conditions to yield single stranded forms of said target sequence, contacting the denatured sample with a set of primers comprising at least two primers wherein at least one primer is capable of hybridizing to one strand of said target sequence and wherein at least one other primer is capable of hybridizing to a strand complementary to said first mentioned strand and wherein said primers are extendable from their 3' termini to form an extension product complementary to the strand to which each of said primers has hybridized and subjecting the sample to conditions to facilitate amplification to generate an amplified product comprising complementary extension products and then detecting for the presence of the amplification product wherein the presence of said amplified product is indicative of said HBV-derived nucleic acid target sequence and then isolating said HBV so detected.

Still a further aspect of the present invention provides a means for identifying expression products such as a peptide, polypeptide or protein useful as immunological markers and/or for generating immunoglobulins for use in diagnosis and/or therapy.

The nucleotide sequences of the HBV identified by the present invention can be used in a variety of recombinant expression systems. Such nucleotide sequences may be conserved or variable. An example of the latter is a modified or new epitope on HBsAg. Such expression systems allow adequate expression of the desired genes, in particular, the gene encoding HBsAg or its variants. Recombinant expression of nucleotide sequences in prokaryotic and/or eukaryotic hosts is well established, with these expressed proteins used in raising immunoglobulins specific to the particular HBsAg's. The immunoglobulins can then be used in assays to detect the presence of the virus. The proteins can also be used to detect the presence of antibodies in patients infected by HBV.

The desired viral sequences are conventionally inserted into a suitable vector before transformation/transfection using standard techniques into mammalian, yeast or insect cell lines for expression. Prokaryotic are preferably used for intermediate cloning steps, but can be efficiently used for high levels of inducible expression of the desired viral sequences.

The particular procedure used to introduce the altered genetic material into the host cell for expression of the viral sequences is not particularly critical. Any of the well known procedures for introducing foreign nucleotide sequences into host cells may be used. These include the use of calciumphosphate transfection, electroporation, liposomes, plasmid vectors, viral vectors and any other well established methods for introducing cloned viral DNA, in particular those identified by the present invention in a test sample that may or may not display viral markers, that may or may not be detected by immune-based diagnostic tests.

The particular vector used to transport the genetic information into the cell is not particularly critical. Any of the conventional vectors used for expression of recombinant proteins in eukaryotic cells may be used.

The expression vector contains a eukaryotic transcription unit or expression cassette that contains all the elements required for the expression of the viral nucleic acid sequences in eukaryotic cells. A typical expression cassette contains a promoter operably linked to the DNA sequence encoding a vital protein and signals required for efficient polyadenylation of the transcribed mRNA.

The expression vector of the present invention will typically contain both prokaryotic sequences that facilitate the cloning of the vector in bacteria (e.g. *E*. *coli*) as well as one or more eukaryotic transcription units that are expressed only in eukaryotic cells, such as mammalian cells. The vector may or may not comprise a eukaryotic replicon. If such replicon is present, then the vector is amplifiable in eukaryotic cells using the appropriate selection marker. If the vector does not comprise a eukaryotic replicon, no episomal amplification is possible. Under this condition, the transfected DNA integrates into the genome of the host cells, with the viral gene expression driven by the co-integrated mammalian promoter on the expression vector.

After the expression vector is introduced into the cells, the transfected cells are cultured under conditions favouring expression of the viral protein and the protein is purified from the culture using standard techniques. A number of standard procedures for purifying proteins can be used, such as, for example, affinity chromatography, size exclusion chromatography and ion exchange chromatography.

In addition to the use of proteins encoded by the desired viral genome, peptides that mimic epitopes specific to the virus can also be used to raise antibodies specific to the virus.

The purified polypeptides or synthetic peptides discussed above are then used to raise antibodies using standard procedures. The immunoglobulins may be used for a variety of applications. For instance, they can be used to assay for the presence of the virus in a test sample or to purify the viral antigen, for example, using affinity chromatography. They may also be used to neutralize the corresponding viral protein, including inhibition of infectiousness of the entire virus. The multitude of techniques available for production and manipulation of various immunoglobulins can thus be readily applied to produce molecules useful for diagnosis and detection of the particular HBV strain in a test sample, that may or may not display viral markers that may or may not be detected by immune-based diagnostic tests.

Antibodies that bind epitopes specific to the desired HBV strain may be produced by a variety of means. The production of non-human monoclonal antibodies (e.g. murine) is well known and may be accomplished by immunizing the animal with a preparation containing the virus or a fragment thereof. Antibody-producing cells obtained from the immunized animals are immortalized and screened using standard techniques.

The immunoglobulins produced as described above can then be used in a variety of diagnostic assays for the presence of HBV strain in a test sample that may or may not display viral markers, that may or may not be detected by immune-based diagnostic tests. For instance, the labelled immunoglobulins can be used in assays involving contacting the immunoglobulins with a test sample suspected of containing the HBV strain that may or may not display viral markers, that may or may not be detected by immune-based diagnostic tests, and detecting whether a complex is formed. A wide variety of labels may be used to couple to the immunoglobulins. A common method of detection is the use of autoradiography with ¹²⁵I or ³⁵S. Non-radioactive labels may be used. Such labels include chemiluminescent agents and enzymes. These non-radioactive labels are often attached by indirect means. Generally, a ligand molecule (e.g. biotin) is covalently bound to the molecule. The ligand then binds to an anti-ligand (e.g. streptavidin) molecule that is either inherently detectable or covalently bound to a signal system, such as a detectable enzyme, a fluorescent compound or a chemiluminescent compound.

The molecules can also be conjugated directly to signal-generating compounds, e.g. by conjugating with an enzyme. Enzymes of interest as labels can be phosphatases or peroxidases.

To detect the presence of the complexes, the mixture will be contacted with a protein capable of binding the Fc region of the immunoglobulins, such as a second antibody, protein A or protein G. The protein is preferably immobilized on a solid surface and the solid surface is washed to remove unbound immunoglobulins specific for the desired virus. Many methods for immobilizing biomolecules can be used. For instance, the solid surface may be a membrane (e.g. nitrocellulose), a microtitre dish (e.g. a polystyrene) or a bead. The desired component may be covalently bound or non-covalently attached through unspecific bonding. The label is then directed using standard techniques.

The recombinantly expressed and purified viral protein or viral lysates may also be used in diagnostic procedures. These procedures typically involve contacting a biological sample (e.g. serum) suspected of containing antibodies to HBV and detecting the immunological reaction. The reaction is preferably detected using labelled proteins as described above.

The method of the present invention is useful, therefore, in identifying conserved regions of the HBV genome which may correspond to conserved regions in HBV proteins such as the HBsAg. The method may also identify variable regions. Consequently, peptides, polypeptides and proteins corresponding to the conserved regions are useful as vaccine components and in diagnostic tests. Similarly, peptides, polypeptides and proteins covering variable regions may be useful for specific vaccines to particular HBV variants or as diagnostic agents.

The present invention provides, therefore, a recombinant or chemically synthetic peptide, polypeptide or protein or derivatives, homologues or analogues thereof comprising a sequence of amino acids encompassing a conserved amino acid sequence between two or more HBV isolates or which varies from HBV wild-type. An HBV wild-type is considered to comprise a composite or consensus nucleotide or amino acid sequence from HBV genotypes E and A through F (13).

A "derivative" includes a part, portion, fragment, section or region of a polypeptide including a polypeptide comprising a single or multiple amino acid substitution, addition and/or deletion. For brevity, a peptide and protein are encompassed by the term "polypeptide".

The present invention extends to chemical analogues of the subject polypeptides including those with modifications to side chains and/or those which incorporate unnatural amino acids. Such chemically modified or synthetic analogues of polypeptides may be more stable for use as diagnostic agents or for use in therapy.

Examples of side chain modifications contemplated by the present invention include modifications of amino groups such as by reductive alkylation by reaction with an aldehyde followed by reduction with NaBH₄; amidination with methylacetimidate; acylation with acetic anhydride; carbamoylation of amino groups with cyanate; trinitrobenzylation of amino groups with 2, 4, 6-trinitrobenzene sulphonic acid (TNBS); acylation of amino groups with succinic anhydride and tetrahydrophthalic anhydride; and pyridoxylation of lysine with pyridoxal-5-phosphate followed by reduction with NaBH₄.

The guanidine group of arginine residues may be modified by the formation of heterocyclic condensation products with reagents such as 2,3-butanedione, phenylglyoxal and glyoxal.

The carboxyl group may be modified by carbodiimide activation *via* O-acylisourea formation followed by subsequent derivitisation, for example, to a corresponding amide.

Sulphydryl groups may be modified by methods such as carboxymethylation with iodoacetic acid or iodoacetamide; performic acid oxidation to cystcic acid; formation of a mixed disulphides with other thiol compounds; reaction with maleimide, maleic anhydride or other substituted malcimide; formation of mercurial derivatives using 4-chloromercuribenzoate, 4-chloromercuriphenylsulphonic acid, phenylmercury chloride, 2-chloromercuri-4-nitrophenol and other mercurials; carbamoylation with cyanate at alkaline pH.

Tryptophan residues may be modified by, for example, oxidation with N-bromosuccinimide or alkylation of the indole ring with 2-hydroxy-5-nitrobenzyl bromide or sulphenyl halides. Tyrosine residues on the other hand, may be altered by nitration with tetranitromethane to form a 3-nitrotyrosine derivative.

Modification of the imidazole ring of a histidine residue may be accomplished by alkylation with iodoacetic acid derivatives or N-carbethoxylation with diethylpyrocarbonate.

Examples of incorporating unnatural amino acids and derivatives during peptide synthesis include, but are not limited to, use of norleucine, 4-amino butyric acid, 4-amino-3-hydroxy-5-phenylpentanoic acid, 6-aminohexanoic acid, t-butylglycine, norvaline, phenylglycine, ornithine, sarcosine, 4-amino-3-hydroxy-6-methylheptanoic acid, 2-thienyl alanine and/or D-isomers of amino acids. A list of unnatural amino acid, contemplated herein is shown in Table 1.

**TABLE 1**

| Non-conventional amino acid | Code | Non-conventional amino acid | Code |
|---|---|---|---|
| α-aminobutyric acid | Abu | L-N-methylalanine | Nmala |
| α-amino--methylbutyrate | Mgabu | L-N-methylarginine | Nmarg |
| aminocyclopropane- | Cpro | L-N-methylasparagine | Nmasn |
| carboxylate | | L-N-methylaspartic acid | Nmasp |
| aminoisobutyric acid | Aib | L-N-methylcysteine | Nmcys |
| aminonorbornyl- | Norb | L-N-methylglutamine | Nmgln |
| carboxylate | | L-N-methylglutamic acid | Nmglu |
| cyclohexylalanine | Chexa | L-N-methylhistidine | Nmhis |
| cyclopentylalanine | Cpen | L-N-methylisolleucine | Nmile |
| D-alanine | Dal | L-N-methylleucine | Nmleu |
| D-arginine | Darg | L-N-methyllysine | Nmlys |
| D-aspartic acid | Dasp | L-N-methylmethionine | Nmmet |
| D-cysteine | Dcys | L-N-methylnorleucine | Nmnle |
| D-glutamine | Dgln | L-N-methylnorvaline | Nmnva |
| D-glutamic acid | Dglu | L-N-methylomithine | Nmorn |
| D-histidine | Dhis | L-N-methylphenylalanine | Nmphe |
| D-isoleucine | Dile | L-N-methylproline | Nmpro |
| D-leucine | Dleu | L-N-methylserine | Nmser |
| D-lysine | Dlys | L-N-methylthreonine | Nmthr |
| D-methionine | Dmet | L-N-methyltryptophan | Nmtrp |
| D-ornithine | Dorn | L-N-methyltyrosine | Nmtyr |
| D-phenylalanine | Dphe | L-N-methylvaline | Nmval |
| D-proline | Dpro | L-N-methylethylglycine | Nmetg |
| D-serine | Dser | L-N-methyl-t-butylglycine | Nmtbug |
| D-tlueonine | Dthr | L-norleucine | Nle |
| D-tryptophan | Dtrp | L-norvaline | Nva |
| D-tyrosine | Dtyr | α-methyl-aminoisobutyrate | Maib |
| D-valine | Dval | α-methyl-Υ-aminobutyrate | Mgabu |
| D-α-methylalanine | Dmala | α-methylcyclohexylalanine | Mchexa |
| D-α-methylarginine | Dmarg | α-methylcylcopentylalanine | Mcpen |
| D-α-methylasparagine | Dmasn | α-methyl-α-napthylalanine | Manap |
| D-α-methylaspartate | Dmasp | a-methylpenicillamine | Mpen |
| D-α-methylcysteine | Dmcys | N-(4-aminobutyl)glycine | Nglu |
| D-α-methylglutamine | Dmgln | N-(2-aminoethyl)glycine | Naeg |
| D-α-methylhistidine | Dmhis | N-(3-aminopropyl)glycine | Norn |
| D-α-methylisolcucine | Dmile | N-amino-α-methylbutyrate | Nmaabu |
| D-α-methylleucine | Dmleu | α-napthylalanine | Anap |
| D-α-methyllysine | Dmlys | N-benzyl glycine | Nphe |
| D-α-methylmethionine | Dmmet | N-(2-carbamylethyl)glycine | Ngln |
| D-α-methylornithine | Dmorn | N-(carbamylmethyl)glycine | Nasn |
| D-α-methylphenylalanine | Dmphe | N-(2-carboxyethyl)glychle | Nglu |
| D-α-methylproline | Dmpro | N-(carboxymethyl)glycine | Nasp |
| D-α-methylserine | Dmser | N-cyclobutylglycine | Ncbut |
| D-α-methylthreonine | Dmthr | N-cycloheptylglycine | Nchep |
| D-α-methyltryptophan | Dmtrp | N-cyclohexylglycine | Nchex |
| D-α-methyltyrosine | Dmty | N-cyclodecylglycine | Ncdec |
| D-α-methylvaline | Dmval | N-cylcododecylglycine | Ncdod |
| D-N-methylalanine | Dnmala | N-cyclooctylglycine | Ncoct |
| D-N-methylarginine | Dnmarg | N-cyclopropylglycine | Ncpro |
| D-N-methylasparagine | Dnmasn | N-cycloundecylglycine | Ncund |
| D-N-methylaspartate | Dnmasp | N-(2,2-diphenylethyl)glycine | Nbhm |
| D-N-methylcysteine | Dnmcys | N-(3,3-diphenylpropyl)glycine | Nbhe |
| D-N-methylglutamine | Dnmgln | N-(3-guanidinopropyl)glycine | Narg |
| D-N-methylglutamate | Dnmglu | N-(1-hydroxyethyl)glycine | Nthr |
| D-N-methylhistidine | Dnmhis | N-(hydroxyethyl))glycine | Nser |
| D-N-methylisoleucine | Dnmile | N-(imidazolylethyl))glycine | Nhis |
| D-N-methylleucine | Dnmleu | N-(3-indolylyethyl)glycine | Nhtrp |
| D-N-methyllysine | Dnmlys | N-methyl-Y-aminobutyrate | Nmgabu |
| N-methylcyclohexylalanine | Nmchexa | D-N-methylmethionine | Dnmmet |
| D-N-methylornithine | Dnmorn | N-methylcyclopentylalanine | Nmcpen |
| N-methylglycine | Nala | D-N-methylphenylalanine | Dnmphe |
| N-methylaminoisobutyrate | Nmaib | D-N-methylproline | Dnmpro |
| N-(1-methylpropyl)glycine | Nile | D-N-methylserine | Dnmser |
| N-(2-methylpropyl)glycine | Nleu | D-N-methylthreonine | Dnmthr |
| D-N-methyltryptophan | Dnmtrp | N-(1-methylethyl)glycine | Nval |
| D-N-methyltyrosine | Dnmtyr | N-methyla-napthylalanine | Nmanap |
| D-N-methylvaline | Dnmval | N-methylpenicillamine | Nmpen |
| Υ-aminobutyric acid | Gabu | N-(*p*-hydroxyphenyl)glycine | Nhtyr |
| L-*t*-butylglycine | Tbug | N-(thiomethyl)glycine | Ncys |
| L-ethylglycine | Etg | penicillamine | Pen |
| L-homophenylalanine | Hphe | L-α-methylalanine | Mala |
| L-α-methylarginine | Marg | L-α-methylasparagine | Masn |
| L-α-methylaspartate | Masp | L-α-methyl-*t*-butylglycine | Mtbug |
| L-α-methylcysteine | Mcys | L-methylethylglycine | Metg |
| L-α-methylglutamine | Mgln | L-α-methylglutamate | Mglu |
| L-α-methylhistidine | Mhis | L-α-methylhomophenylalanine | Mhphe |
| L-α-methylisoleucine | Mile | N-(2-methylthioethyl)glycine | Nmet |
| L-α-methylleucine | Mleu | L-α-methyllysine | Mlys |
| L-α-methylmethionine | Mmet | L-α-methylnorleucine | Mnle |
| L-α-methylnorvaline | Mnva | L-α-methylornithine | Morn |
| L-α-methylphenylalanine | Mphe | L-α-methylproline | Mpro |
| L-α-methylserine | Mser | L-α-methylthreonine | Mthr |
| L-α-methyltryptophan | Mtrp | L-α-methyltyrosine | Mtyr |
| L-α-methylvaline | Mval | L-N-methylhomophenylalani | Nmhphe |
| N-(N-(2,2-diphenylethyl) | Nnbhm | N-(N-(3,3-diphenylpropyl) | Nnbhe |
| carbamylmethyl)glycine | | carbamylmethyl)glycine | |
| 1-carboxy-1-(2,2-diphenyl- | Nmbc | | |
| ethyl amino)cyclopropane | | | |

The polypeptides of the present invention may be used in a pharmaceutical composition to vaccinate against an HBV or an HBV variant. The pharmaceutical composition contains the subject polypeptides as well as one or more pharmaceutically acceptable carriers and/or diluents.

Pharmaceutically acceptable carriers and/or diluents include any and all solvents, dispersion media, coatings, antibacterial and antifungal agents, isotonic and absorption delaying agents and the like. The use of such media and agents for pharmaceutical active substances is well known in the art. Except insofar as any conventional media or agent is incompatible with the active ingredient, use thereof in the therapeutic compositions is contemplated. Supplementary active ingredients can also be incorporated into the compositions.

The pharmaceutical composition may also comprise genetic molecules such as a vector capable of transfecting target cells where the vector carries a nucleic acid molecule encoding the subject polypeptide or encoding a ribozyme or antisense molecule to an HBV-derived genetic sequence. The vector may, for example, be a viral, bacterial or eukaryotic vector.

As stated above, the present invention further extends to antibodies to the subject polypeptide. The antibodies may be monoclonal or polyclonal. Such antibodies are useful for therapy and in diagnostic assays.

Another aspect of the present invention contemplates a method for detecting an HBV in a biological sample from a subject said method comprising contacting said biological sample with an antibody specific for an HBV or its variants for a time and under conditions sufficient for an antibody-HBV complex to form, and then detecting said complex.

The presence of HBV may be accomplished in any number of ways such as by Western blotting and ELISA procedures. A wide range of immunoassay techniques are available as can be seen by reference to U.S. Patent Nos. 4,016,043, 4,424,279 and 4,018,653. These include both single-site and two-site or "sandwich" assays of the non-competitive types, as well as in the traditional competitive binding assays. These assays also include direct binding of a labelled antibody to a target.

Sandwich assays are among the most useful and commonly used assays and are favoured for use in the present invention. A number of variations of the sandwich assay technique exist, and all are intended to be encompassed by the present invention. Briefly, in a typical forward assay, an unlabelled antibody is immobilized on a solid substrate and the sample to be tested brought into contact with the bound molecule. After a suitable period of incubation, for a period of time sufficient to allow formation of an antibody-antigen complex, a second antibody specific to the antigen, labelled with a reporter molecule capable of producing a detectable signal is then added and incubated, allowing time sufficient for the formation of another complex of antibody-antigen-labelled antibody. Any unreacted material is washed away, and the presence of the antigen is determined by observation of a signal produced by the reporter molecule. The results may either be qualitative, by simple observation of the visible signal, or may be quantitated by comparing with a control ample containing known amounts of hapten. Variations on the forward assay include a simultaneous assay, in which both sample and labelled antibody are added simultaneously to the bound antibody. These techniques are well known to those skilled in the art, including any minor variations as will be readily apparent. In accordance with the present invention, the sample is one which might contain an HBV or an HBV-derived polypeptide including a cell extract, tissue biopsy or possibly serum, saliva, mucosal secretions, lymph, tissue fluid and respiratory fluid. The sample is, therefore, generally a biological sample comprising biological fluid but also extends to fermentation fluid and supernatant fluid such as from a cell culture.

In a typical forward sandwich assay, a first antibody having specificity for the HBV or antigenic parts thereof, is either covalently or passively bound to a solid surface. The solid surface is typically glass or a polymer, the most commonly used polymers being cellulose, polyacrylamide, nylon, polystyrene, polyvinyl chloride or polypropylene. The solid supports may be in the form of tubes, beads, discs of microplates, or any other surface suitable for conducting an immunoassay. The binding processes are well-known in the art and generally consist of cross-linking covalently binding or physically adsorbing, the polymer-antibody complex is washed in preparation for the test sample. An aliquot of the sample to be tested is then added to the solid phase complex and incubated for a period of time sufficient (e.g. 2-40 minutes or overnight if more convenient) and under suitable conditions (e.g. from room temperature to about 37°C such as about 25°C) to allow binding of any subunit present in the antibody. Following the incubation period, the antibody subunit solid phase is washed and dried and incubated with a second antibody specific for a portion of the hapten. The second antibody is linked to a reporter molecule which is used to indicate the binding of the second antibody to the hapten.

An alternative method involves immobilizing the target molecules in the biological sample and then exposing the immobilized target to specific antibody which may or may not be labelled with a reporter molecule. Depending on the amount of target and the strength of the reporter molecule signal, a bound target may be detectable by direct labelling with the antibody.

Alternatively, a second labelled antibody, specific to the first antibody is exposed to the target-first antibody complex to form a target-first antibody-second antibody tertiary complex. The complex is detected by the signal emitted by the reporter molecule.

By "reporter molecule", as used in the present specification, is meant a molecule which, by its chemical nature, provides an analytically identifiable signal which allows the detection of antigen-bound antibody. Detection may be either qualitative or quantitative. The most commonly used reporter molecules in this type of assay are either enzymes, fluorophores or radionuclide containing molecules (i.e. radioisotopes) and chemiluminescent molecules.

In the case of an enzyme immunoassay, an enzyme is conjugated to the second antibody, generally by means of glutaraldehyde or periodate. As will be readily recognized, however, a wide variety of different conjugation techniques exist, which are readily available to the skilled artisan. Commonly used enzymes include horseradish peroxidase, glucose oxidase, -galactosidase and alkaline phosphatase, amongst others. The substrates to be used with the specific enzymes are generally chosen for the production, upon hydrolysis by the corresponding enzyme, of a detectable color change. Examples of suitable enzymes include alkaline phosphatase and peroxidase. It is also possible to employ fluorogenic substrates, which yield a fluorescent product rather than the chromogenic substrates noted above. In all cases, the enzyme-labelled antibody is added to the first antibody hapten complex, allowed to bind, and then the excess reagent is washed away. A solution containing the appropriate substrate is then added to the complex of antibody-antigen-antibody. The substrate will react with the enzyme linked to the second antibody, giving a qualitative visual signal, which may be further quantitated, usually spectrophotometrically, to give an indication of the amount of hapten which was present in the sample. "Reporter molecule" also extends to use of cell agglutination or inhibition of agglutination such as red blood cells on latex beads, and the like.

Alternately, fluorescent compounds, such as fluorecein and rhodamine, may be chemically coupled to antibodies without altering their binding capacity. When activated by illumination with light of a particular wavelength, the fluorochrome-labelled antibody adsorbs the light energy, inducing a state to excitability in the molecule, followed by emission of the light at a characteristic color visually detectable with a light microscope. As in the EIA, the fluorescent labelled antibody is allowed to bind to the first antibody-hapten complex. After washing off the unbound reagent, the remaining tertiary complex is then exposed to the light of the appropriate wavelength, the fluorescence observed indicates the presence of the hapten of interest. Immunofluorescene and EIA techniques are both very well established in the art and are particularly preferred for the present method. However, other reporter molecules, such as radioisotope, chemiluminescent or bioluminescent molecules, may also be employed.

The present invention is further described by the following non-limiting Examples.

### EXAMPLE 1

### Detection and identification of HBV surface antigen mutants in Singapore adults and vaccinated infants with high anti-HBs levels but negative for surface antigen

The presence of HBV DNA, in Singapore adults and vaccinated infants negative for HBsAg, is investigated by polymerase chain reaction (PCR) amplification using primers oligonucleotides provided in the present invention. Sixty-three adults and 15 vaccinated infants (aged below 15 years, and with recombinant vaccine) from different ethnic groups, who are tested negative for HBsAg by four independent commercial immuno-based diagnostic kits, are selected (Table 2). They are all positive for total antibodies to HBV core antigen (anti-HBc) (Corzyme, Abbott Laboratories, USA). All are also positive for anti-HBs (Ausab, Abbott Laboratories, USA) (Table 2). DNA from 100 µl of serum is extracted by proteinase K treatment, followed by chloroform/phenol extraction and ethanol precipitation. Oligonucleotides provided in the present invention are utilized in polymerase chain reaction (PCR) amplification with *Pfu* DNA polymerase (Stratagene, USA). Amplification was performed in 35 cycles, each of them consisting of denaturation at 94°C (1 min), annealing at 50°C (2 min) and extension at 73°C (3 min). Results indicate that HBV DNA is amplified using the primer oligonucleotides provided in the present invention in three of the 15 infants (20%) and eight of the 63 (13%) adults. The identification of HBsAg mutants in this group of HBsAg negative infants represented a distinct situation, as compared with our previous report on the detection of HBsAg mutants in vaccinated Singapore infants who were tested positive to HBsAg. HBV DNA is amplified using the primer oligonucleotides provided in the present invention in all samples in this group of vaccinated infants.

Direct sequencing of the amplified DNA fragments revealed mutations at various positions of the MHR in these samples (Table 2). These include the most common vaccine escape G145R in six cases. There are also three cases here with the G130D mutation (Table 2) that is recently found in one case report to be associated with lamivudine therapy. In addition, the T131N mutation is identified in four cases. Unlike the reported genotype-associated T131N mutation which always carries a concurrent T114S on HBsAg, the T131N mutation identified using the primer oligonucleotides provided in the present invention has a wild-type S (serine) at residue 114. This in turn suggests that it could be a variant capable of escaping detection.

Those skilled in the art will appreciate that the invention described herein is susceptible to variations and modifications other than those specifically described. It is to be understood that the invention includes all such variations and modifications. The invention also includes all of the steps, features, compositions and compounds referred to or indicated in this specification, individually or collectively, and any and all combinations of any two or more of said steps or features.

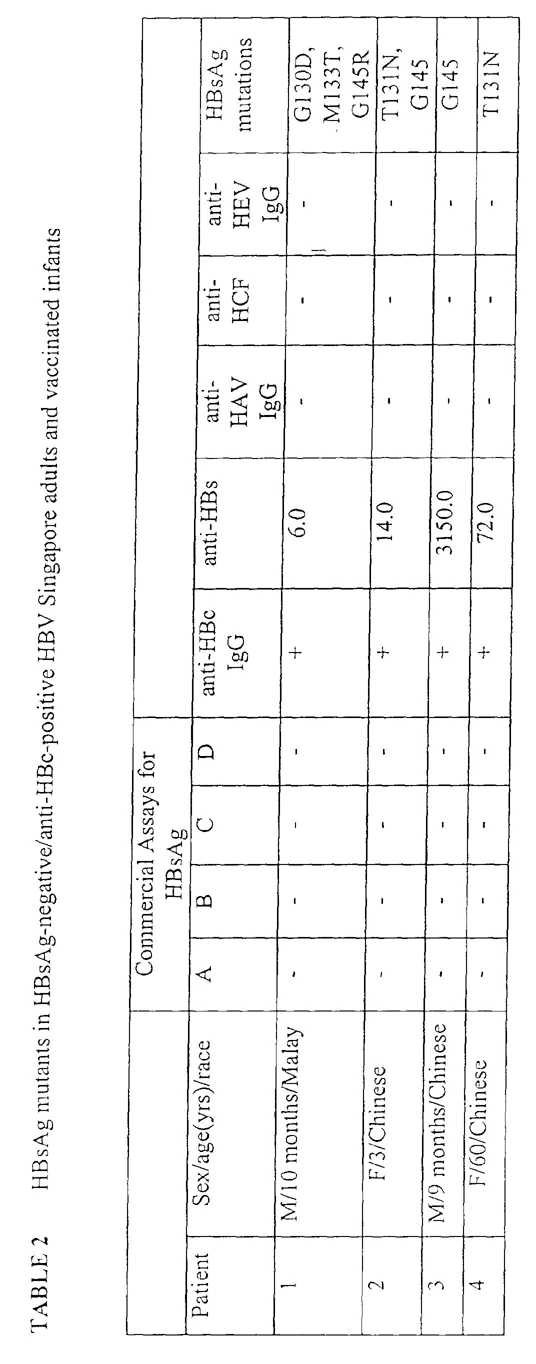

### BIBLIOGRAPHY

1. Carman *et al*. *Gastroenterology* **102**:711-719, 1992.
2. Carman *et al*. *Lancet* **336**:325-329, 1990.
3. Okamoto *et al. Paediatric Research* **32**:264-268, 1992.
4. McMahon *et al. Hepatology* **15**:757-766, 1992.
5. Fujii *et al*. *Biochem. Biophys. Res. Commun*. **184**:1152-1157, 1992.
6. Harrison *et al. J*. *Hepatol*. **13**:5105-5107, 1991.
7. Chen *et al. FEBS Lett*. **453**:237-242, 1999.
8. Altschul *et al., Nucl*. *Acids Res.* **25**:3389. 1997.
9. Ausubel *et al.,* "Current Protocols in Molecular Biology" John Wiley & Sons Inc, 1994-1998, Chapter 15.
10. Bonner and Laskey *Eur. J. Biochem. 46:* 83, 1974.
11. Marmur and Doty *J*. *Mol. Biol*. *5:* 109, 1962.
12. Oon *et al. Vaccine* **13**:699-702, 1999.

## Claims

1. A method for detecting an HBV-derived nucleic acid target sequence in a sample, said method comprising subjecting the sample to denaturing conditions to yield single stranded forms of said target sequence, contacting the denatured sample with a set of primers comprising at least two primers wherein at least one primer is capable of hybridizing to one strand of said target sequence and wherein at least one other primer is capable of hybridizing to a strand complementary to said first mentioned strand and wherein said primers are extendable from their 3' termini to form an extension product complementary to the strand to which each of said primers has hybridized and subjecting the sample to conditions to facilitate amplification to generate an amplified product comprising complementary extension products and then detecting for the presence of the amplification product wherein the presence of said amplified product is indicative of said HBV-derived nucleic acid target sequence.

2. A method according to Claim 1 wherein the primers hybridize to regions corresponding to or flanking a nucleotide sequence within or part of the nucleotide sequence encoding HBsAg.

3. A method according to Claim 2 wherein the primers hybridize to regions corresponding to or flanking a conserved nucleotide sequence within a part of the nucleotide sequence encoding HBsAg.

4. A method according to Claim 1 or 2 or 3 further comprising first subjecting the sample to reverse transcription conditions to yield single or double stranded cDNA molecules from HBV-derived mRNA.

5. A method according to Claim 1 wherein one of said primers is labelled with a reporter molecule capable of giving an identifiable signal and the other of said primers is labelled with a capturable moiety.

6. A method for detecting an HBV-derived DNA target sequence in a sample, said method comprising introducing said sample to a reaction vessel having a primer immobilized to a solid support and a second primer in solution phase wherein both primers are capable of hybridizing to a complementary nucleotide sequence on complementary single strands of HBV-derived DNA in a region within, proximal or adjacent to a conserved region on the HBV genome and wherein the solution phase primer is labelled with a reporter molecule capable of giving an identifiable signal, subjecting the reaction vessel to conditions to facilitate amplification and then detecting the presence of the identifiable signal wherein the presence of said signal is indicative of the presence of HBV-derived DNA.

7. A method for detecting one or more HBV-derived DNA target sequences from the same or different strains or variants of HBV, said method comprising contacting a sample putatively comprising HBV-derived DNA in single stranded form with two or more primers immobilized individually or in an array to a solid support in a reaction vessel wherein the reaction vessel further comprises solution phase primers having an partner immobilized to the solid support wherein the immobilized primer and its solution phase partner primer are capable of amplifying under amplifying conditions a region of HBV-derived DNA wherein the solution phase primer carries a reporter molecule capable of giving an identifiable signal such that the presence of a signal at a defined location on the solid support enables the identification of a particular HBV isolate or variant.

8. An array of two or more oligonucleotide primers immobilized to a solid support, said primers capable of hybridizing to a complementary nucleotide sequence from HBV-derived DNA.

9. An array according to Claim 8 wherein the array is defined by the formula a₁a₂ ... aₙ, having coordinates on the matrix (x₁y₁), (x₂y₂) ... (xₙyₙ) wherein a₁a₂ ... aₙ represent the same or different oligonucleotides totalling n oligonucleotides and each oligonucleotide is definable by grid coordinates (x₁y₁), (x₂y₂) ... (xₙyₙ) and wherein the oligonucleotides have amplification partners defined by b₁b₁ ... bₙ such that oligonucleotides a₁b₁, a₂b₂ ... aₙbₙ are capable of hybridizing to complementary strands of HBV-derived DNA wherein the intervening DNA comprises a sequence of nucleotides conserved between two or more HBV variants, such as HBsAg variants, wherein said matrix is useful for detecting particular HBV agents defined by carrying the conserved amplification region of said HBV-derived DNA.

10. A method according to Claim 1 wherein the primers are selected from <400>1 and <400>2 or primers having at least 70% similarity thereto or primers capable of hybridizing to <400> 1 or <400>2 under low stringency conditions.

11. An HBV variant, generally in isolated form, which HBV variant is identified by a method of detecting an HBV-derived nucleic acid target sequence, said method comprising subjecting the sample to denaturing conditions to yield single stranded forms of said target sequence, contacting the denatured sample with a set of primers comprising at least two primers wherein at least one primer is capable of hybridizing to one strand of said target sequence and wherein at least one other primer is capable of hybridizing to a strand complementary to said first mentioned strand and wherein said primers are extendable from their 3' termini to form an extension product complementary to the strand to which each of said primers has hybridized and subjecting the sample to conditions to facilitate amplification to generate an amplified product comprising complementary extension products and then detecting for the presence of the amplification product wherein the presence of said amplified product is indicative of said HBV-derived nucleic acid target sequence and then isolating said HBV so detected.

12. A means for identifying expression products such as a peptide, polypeptide or protein useful as immunological markers and/or for generating immunoglobulins for use in diagnosis and/or therapy.
